# EUROPEAN PATENT SPECIFICATION

(11) **EP 3 931 197 B1**
(45) Date of publication and mention of the grant of the patent: **24.04.2024**
(21) Application number: 20707238.0
(22) Date of filing: 25.02.2020
(51) Int. Cl.: C07D 487/04

(54) **PROCESS FOR THE PREPARATION OF (6S)-3-[(4S)-4-CYANO-2-OXO-PYRROLIDIN-1-YL]-6-METHYL-N-(3,4,5-TRIFLUOROPHENYL)-6,7-DIHYDRO-4H-PYRAZOLO[1,5-A]PYRAZINE-5-CARBOXAMIDE**
VERFAHREN ZUR HERSTELLUNG VON (6S)-3-[(4S)-4-CYANO-2-OXO-PYRROLIDIN-1-YL]-6-METHYL-N-(3,4,5-TRIFLUOROPHENYL)-6,7-DIHYDRO-4H-PYRAZOLO[1,5-A]PYRAZIN-5-CARBOXAMID
PROCÉDÉ DE PRÉPARATION DE (6S)-3-[(4S)-4-CYANO-2-OXO-PYRROLIDIN-1-YL]-6-MÉTHYL-N-(3,4,5-TRIFLUOROPHÉNYL)-6,7-DIHYDRO-4 H-PYRAZOLO [1,5-A] PYRAZINE-5-CARBOXAMIDE

(30) Priority: 26.02.2019 WO PCT/CN2019/076126
(43) Date of publication of application: 05.01.2022
(73) Proprietor: F. Hoffmann-La Roche AG, 4070 Basel (CH)
(72) Inventor: CHEN, Weichun, Shanghai, 201203 (CN); WANG, Lin, Shanghai, 201203 (CN); ZHAO, Qiwu, Shanghai, 201203 (CN)
(74) Representative: Belkacemi, Doria
(86) International application number: PCT/EP2020/054806
(87) International publication number: WO 2020/173891

(56) References cited:
- WO-A1-2014/210354
- WO-A1-2016/113273
- WO-A1-2018/011100
- DATABASE REGISTRY [Online] CHEMICAL ABSTRACTS SERVICE, COLUMBUS, OHIO, US; 2 June 2019 (2019-06-02), XP002798444, Database accession no. 2322934-47-2

## Description

The present invention relates to a process for the preparation of (6S)-3-[(4S)-4-cyano-2-oxo-pyrrolidin-1-yl]-6-methyl-N-(3,4,5-trifluorophenyl)-6,7-dihydro-4H-pyrazolo [1,5-a]pyrazine-5-carboxamide (compound (I)), 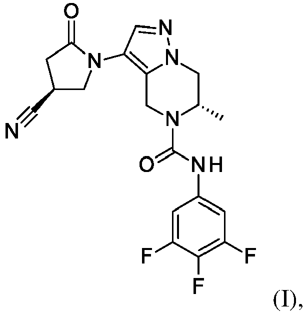 or pharmaceutically acceptable salt thereof, which is useful for prophylaxis and treatment of a viral disease in a patient relating to hepatitis B infection or a disease caused by hepatitis B infection.

### BACKGROUND OF THE INVENTION

The relevant synthetic approach of compound (I) was disclosed as Example 240 in patent WO2016113273, however it is not suitable for commercial process due to the following issues:
1. chiral separation was required;
2. excessive and toxic I₂ was not removed during the synthesis of tert-butyl (6S)-3-iodo-6-methyl-6,7-dihydro-4H-pyrazolo[1,5-a]pyrazine-5-carboxylate, which makes it highly dangerous for large scale manufacture;
3. if chiral starting material ((3S)-5-oxopyrrolidine-3-carbonitrile) was used, the chirality is susceptible to the Ullmann reaction condition and intent to undergo epimerization.

Based on the issues above, one object of the invention therefore is to find an alternative efficient synthetic approach which can be applied on a technical scale and/or result in obtaining the product in a higher yield and/or desired purity as well as required chiral configuration. Addressing any of the issues mentioned above is also one of the objects of the invention.

Furthermore, the advantage of current process of this invention over the previous one disclosed in the prior art are:
1. no chiral separation is needed, and the synthesis of chiral starting material ((3S)-5-oxopyrrolidine-3-carbonitrile) is firstly reported in current invention, which can be easily made in large scale and therefore shortens the whole process;
2. excessive and toxic I₂ was removed in step 1) with reducing agent compared to prior art, the amount of which was then carfully controlled to avoid loss of product;
3. Ullmann reaction condition was carefully optimized to keep the chirality of (3S)-5-oxopyrrolidine-3-carbonitrile moiety unchanged throughout the reaction;
4. the de-BOC reaction was performed in an environment friendly condition.

### DETAILED DESCRIPTION OF THE INVENTION

### DEFINITIONS

The term "pharmaceutically acceptable salt" refers to conventional acid-addition salts or base-addition salts that retain the biological effectiveness and properties of the compounds of formula I and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et al., Organic Process Research & Development 2000, 4, 427-435; or in Ansel, H., et al., In: Pharmaceutical Dosage Forms and Drug Delivery Systems, 6th ed. (1995), pp. 196 and 1456-1457.

### ABBREVIATION

- ACN: Acetonitrile
- eq: Equivalent
- DBU: 1,8-Diazabicyclo[5.4.0]undec-7-ene
- DCM: Dichloromethane
- DIPEA: N,N-Diisopropylethylamine
- DMEDA: N,N'-Dimethyl-1,2-ethanediamine
- IPA: Isopropyl alcohol
- IPAc: Isopropyl acetate
- MeTHF: 2-Methyl tetrahydrofuran
- MTBE: Methyl tert-butyl ether
- NIS: N-Iodosuccinimide
- TEA: Triethylamine
- TFA: Trifluoroacetic acid
- TFAA: Trifluoroacetic anhydride
- V: volume
- wt%: weight percent

The present invention provides the process for preparing the compound (I) as outlined in the **Scheme 1** and compound (IV) as outlined in the **Scheme 2.**

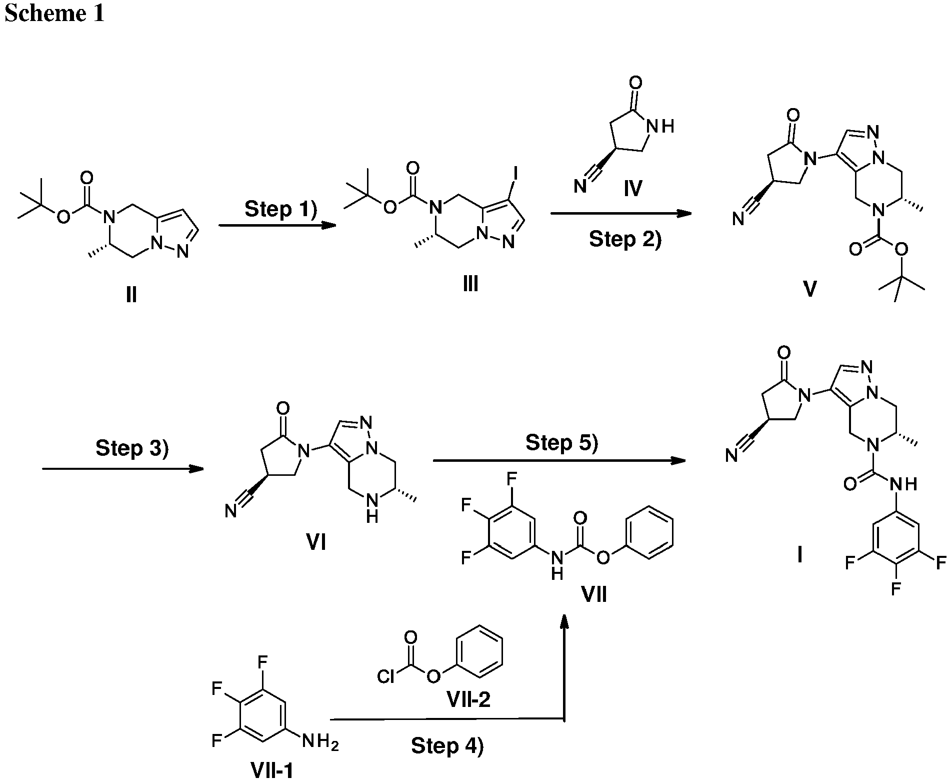

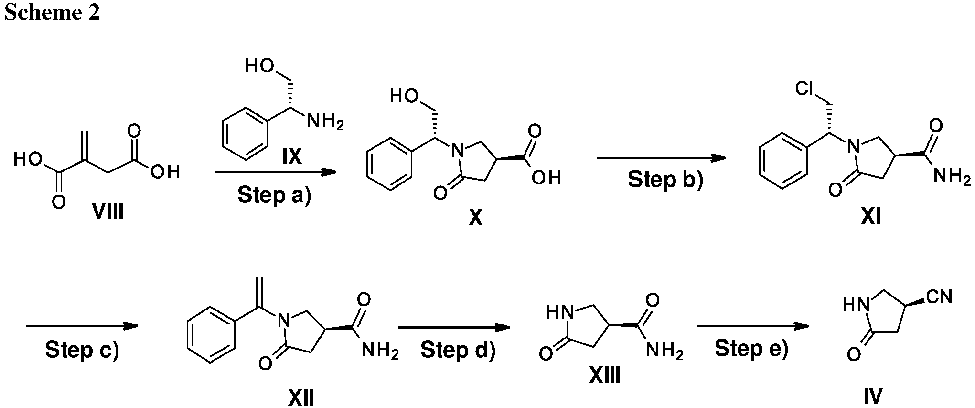

The synthesis of compound (I) comprises one or more of the following steps:
step 1) the formation of compound (III), 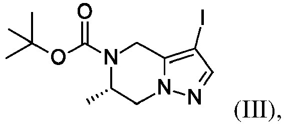 via iodization reaction of compound (II), 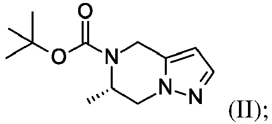
step 2) the formation of compound (V), 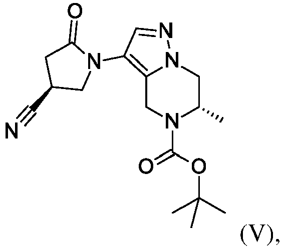 via Ullmann reaction between compound (III) and compound (IV), 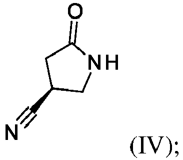
step 3) the formation of compound (VI), 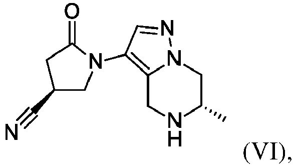 via de-protection of compound (V);
step 4) the formation of compound (VII), 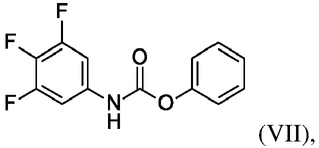 via the reaction between 3,4,5-trifluoroaniline and phenyl carbonochloridate;
step 5) the formation of compound (I), 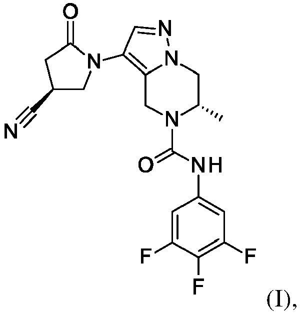 via the substitution reaction between compound (VI) and compound (VII).

A detailed description of synthesis of compound (I) in present invention of process steps is as following:
Step 1) the formation of compound (III), 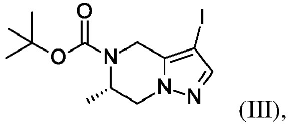 via iodization reaction of compound (II),

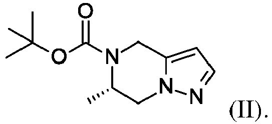

The formation of compound (III) is usually performed in the presence of a suitable iodization reagent and a suitable organic solvent. The conversion as a rule is performed under room temperature or heating condition.

The suitable iodization reagent is selected from NIS and I₂, particularly the reagent is NIS. The excessive I₂ formed during the reaction due to the use of iodization reagent was removed by addition of Na₂SO₃ with amount of 0.4-0.8 eq., particularly 0.55-0.6 eq.

The suitable organic solvent is selected from DCM, THF, ACN and MeTHF, particularly the organic solvent is ACN.

The iodization reaction as a rule is performed at 10~60 °C, particularly at 25~30 °C.

The reaction was quenched by Na₂SO₃ to remove excessive I₂ formed during the reaction, which is very important for the safety of large scale manufacture. However, it was found during the test that excessive Na₂SO₃ will reduce the product and convert compound (III) back to compound (II), while inadequate Na₂SO₃ can not efficiently quench the reaction. A series of tests were done and summarized in the table below, it was concluded that 0.55-0.6 eq. of Na₂SO₃ can meet the above criteria and therefore considered as the optimal condition.

**Table 1. tests on the impact of the amount of Na₂SO₃**

| **Test No.** | **Eq. of Na₂SO₃** | **Results** |
|---|---|---|
| 1 | 0.4 | The reaction was not quenched sufficiently, the color of the product was dark |
| 2 | 0.55 | 0.2% reduction impurity was found once workup was done |
| 3 | 0.6 | 0.5% reduction impurity was found once workup was done |
| 4 | 0.8 | 2% reduction impurity was found once workup was done |

Step 2) the formation of compound (V), 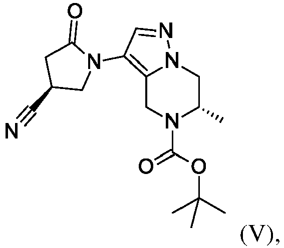 via Ullmann reaction between compound (III) and compound (IV),

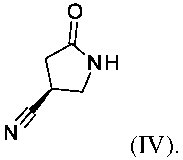

The formation of compound (V) is usually performed in the presence of a suitable base, a suitable catalyst, a suitable ligand and a suitable organic solvent. The conversion as a rule is performed under a heating condition.

The suitable base is selected from K₂CO₃, K₃PO₄ and Cs₂CO₃, particularly the base is K₂CO₃.

The suitable catalyst is CuI and the amount of catalyst is 0.05-0.5eq, particularly 0.1 eq.; the suitable ligand is DMEDA and the amount of ligand is 0.2-2.0 eq., particularly 1.0 eq.

The suitable organic solvent is selected from 1,4-Dioxane, ACN, Toluene, THF and MeTHF, particularly the organic solvent is THF.

The dehydration reaction as a rule is performed at 60°C~120°C, particularly at 70°C~75°C.

The chiral center next to cyano group of compound (V) is intent to epimerize under the Ullmann reaction condition, therefore it is necessary to optimize the condition to keep the chirality of compound (V) steady during the reaction. However, it is very complicated to manage reaction parameters, such as base, solvent, catalyst and ligand loading, reaction time, to achieve the best conversion as well as the chiral purity. The following tests were performed to solve the above problem.
i) The impact of bases on the conversion and chiral

**Table 2. Test of the bases**

| Test No. | Reaction conditions | Check point (reaction time) | Conversion | Chiral purity |
|---|---|---|---|---|
| 1 | | 4h | 50% | 97.2% |
| | 0.5 g compound (III)/1.5 eq. compound (IV)/0.1eq. CuI/0.2 eq. DMEDA/2eq. | 6h | 50% | 97.6% |
| | | 21h | 74% | 95.7% |
| | | 28h | 77% | 93.4% |
| | **K₃PO₄**/MeCN (10V)/50~55°C | 46h | 88% | 92.0% |
| 2 | 1) 0.5 g compound (III)/1.5 eq. compound (IV)/0.1eq CuI/0.2 eq DMEDA/2eq. **K₂CO₃**/ MeCN (10V)/ 50-55 °C | 5h | 43.7% | 97.5% |
| | | 26h | 44.2% | 97.5% |
| | 2) another batch of 0.1 eq CuI/0.2 eq DMEDA were added | 28.5h | 68.7% | - |
| | | 46.5h | 70% | - |
| | 3) another batch of 0.2 eq CuI/0.4 eq DMEDA were added | 51.5h | >99% | 97.8% |
| | | 73.5h | >99% | 97.5% |
| 3 | 0.5 g compound (III)/1.5 eq. compound (IV)/0.1 eq. CuI/0.2 eq. DMEDA/2eq. **Cs₂CO₃**/ MeCN (10V)/50~55°C | 5h | 4% | - |
| | | 21h | 8% | 50% |

Based on above test, Cs₂CO₃ can not achieve enough conversion; K₃PO₄ may achieve higher conversion but chiral purity dropped in the meantime; although K₂CO₃ did not achieve satisfactory conversion at beginning, but the conversion can be boosted by adding more catalyst and ligand, and chiral purity was stable and excellent throughout the whole test. Therefore, K₂CO₃ was selected as the suitable base.
ii) The impact of solvents on the conversion and chiral purity

**Table 3. Test of the solvents**

| Test No. | Reaction conditions | Check point (reaction time) | Conversion | Chiral purity |
|---|---|---|---|---|
| 1 | 0.5 g compound (III)/1.5 eq. compound (IV)/0.1 eq. CuI/0.2 eq. DMEDA/2eq. K₂CO₃/ **MeTHF** (10V)/ 50~55°C | 18h | 20% | 99% |
| 2 | 1) 0.5g compound (III)/ 1.5 eq. compound (IV)/0.1eq CuI/0.2 eq DMEDA/ 2 eq. K₂CO₃/ **THF** (10V)/50~55 °C | 18h | 30% | 98% |
| | 2) another batch of 0.2 eq CuI/0.4 eq DMEDA were added | 25h | 89.6% | 98.1% |
| | 3) another batch of 0.1 eq CuI/0.2 eq DMEDA were added | 43h | 97.5% | 97.5% |
| 3 | 0.5 g compound (III)/1.5 eq. compound (IV)/0.1 eq. CuI/0.2 eq. DMEDA/2eq. | 0 | - | - |
| | K₂CO₃/**Toluene** (10V)/50~55°C | | | |

Based on above tests, the test with THF (Table 3, Test No. 2, item 1)) has higher conversion than the test with MeTHF (Table 3, Test No. 1) at 18h. By comparing the tests with THF (Table 3, Test No. 2) and MeCN (Table 2, Test No. 2), when the final amount of CuI and DMEDA were reached same, the test with THF took shorter reaction time to achieve similar conversion than the test with MeCN. Therefore, THF was selected as the suitable solvent.
iii) The impact of catalyst and ligand loading on the conversion and chiral purity

**Table 4. Test of catalyst and ligand loading**

| Test No. | Reaction conditions | Check point (reaction time) | Conversion | Chiral purity |
|---|---|---|---|---|
| 1 | 1) 50g/0.1 eq CuI*/**0.2 eq** DMEDA, 3 eq. K₂CO₃ powder, THF (8V), 68 °C, | 1h | 42.2% | |
| | 2) another batch of **1.8 eq** DMEDA dissolved in THF (2V) was added dropwise over 2h at 68 °C, then refluxing | 3h | 54.4% | |
| | | 18h | 91.3% | 99.4% |
| 2 | 1) 50 g/0.1eq CuI*/**1 eq** DMEDA, 3 eq. K₂CO₃ powder, THF (8V), 68 °C | 1h | 27% | |
| | 2) another batch of **1 eq** DMEDA dissolved in THF (2V) was added dropwise over 2h at 68 °C, then refluxing | 3h | 33% | |
| | | 18h | 88.2% | 98.8% |
| 3 | 1) 50g/ 0.1 eq CuI*/**0.2 eq** DMEDA, 3 eq. K₂CO₃ powder, THF (8V), 68 °C | 1h | 35.3% | |
| | 2) another batch of **0.8 eq** | 3h | 50.4% | 98.8% |
| | DMEDA dissolved in THF (2V) was added dropwise over 2h at 68 °C, then refluxing | 20h | 85% | |
| 4 | 1) 5g/ 0.1 eq CuI*/0.2 eq DMEDA, 3 eq. K₂CO₃ powder, THF (10V), 68 °C, | 3h | 69% | |
| | 2) 0.3 eq DMEDA added | 5h | 80% | |
| | 3) 0.3 eq DMEDA added | 18h | 87.3% | 99.7% |
| 5 | 1) 50g/0.1 eq CuI*/0.2 eq DMEDA, 3 eq. K₂CO₃ powder, THF (8V), 68 °C | 1h | 64.5% | |
| | 2) **0.8 eq** DMEDA dissolved in | 3h | 80% | 99.5% |
| | THF (2V) was added dropwise over 2h at 68 °C, then refluxing | 20h | 91.7% | |

| | | | | |
|---|---|---|---|---|
| * CuI in this test was obtained from Sundia, which has much higher activity than those previously used. | | | | |

Based on the test No. 1-3 above, the ligand loading does not impact on the conversion and chiral purity too much, and it is finally determined as 1.0 eq. as the optimal ligand loading due to cost reason. By comparing the test of No. 2 in Table 1, No. 2 in Table 3, No. 4 and 5 in Table 4, it is clear that increasing the catalyst loading could increase the conversion and shorten the reaction time, however 0.1 eq. of catalyst loading is already enough to reach satisfactory conversion and chiral purity. Meanwhile, minimum catalyst loading could be environmental friendly and beneficial during heavy metal removal.

Step 3) the formation of compound (VI), 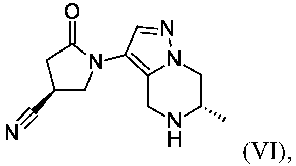 via de-protection of compound (V).

The formation of compound (VI) is usually performed in the presence of a suitable acid and a suitable solvent. The conversion as a rule is performed under room temperature or a heating condition.

The suitable acid is selected from H₃PO₄, NH₄Cl, TFA and acetic acid, particularly the acid is acetic acid and the amount of acid is 1∼3 eq., particularly 1.5-2 eq.

The suitable solvent is selected from DCM, EtOH, water and toluene, particularly the solvent is water.

The de-protection reaction as a rule is performed at room temperature or 60~100 °C, particularly at 90∼95 °C.

Step 4) the formation of compound (VII), 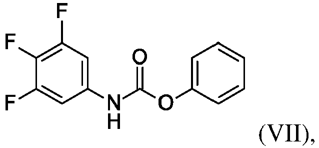 via the reaction between 3,4,5-trifluoroaniline and phenyl carbonochloridate.

The formation of compound (VII) is usually performed in the presence of a suitable base and a suitable organic solvent. The conversion as a rule is performed under a cooling condition.

The suitable base is selected from K₂CO₃, KHCO₃, NaHCO₃ and Na₂CO₃, particularly the base is NaHCO₃.

The suitable organic solvent is selected from MTBE, IPAc, Dioxane, MeTHF and THF, particularly the organic solvent is THF.

The reaction as a rule is performed at -10-10 °C, particularly at -5-0 °C.

Step 5) the formation of compound (I), 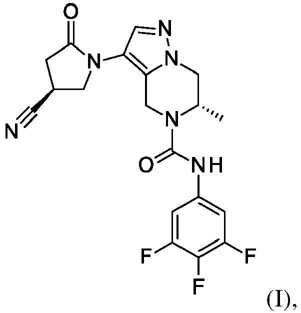 via the substitution reaction between compound (VI) and compound (VII).

The formation of compound (I) is usually performed in the presence of a suitable base and a suitable organic solvent. The conversion as a rule is performed under a heating condition.

The suitable base is selected from K₂CO₃, K₃PO₄, DIPEA and TEA, particularly the base is DIPEA.

The suitable organic solvent is selected from MTBE, EA, IPAc, MeTHF and a mixture of IPAc/acetone, particularly the organic solvent is a mixture of IPAc/acetone.

The substitution reaction as a rule is performed at 30~80 °C, particularly at 45~50 °C.

The synthesis of compound (IV) comprises one or more of the following steps:
step a) the formation of compound (X), 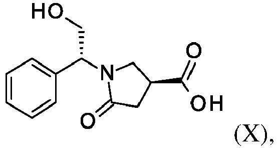 via cyclization reaction between compound (VIII), 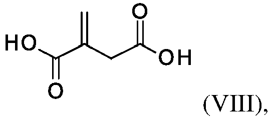 and compound (IX), 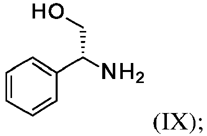
step b) the formation of compound (XI), 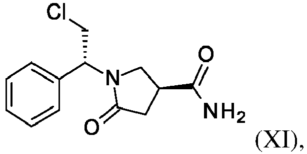 via substitution of compound (X);
step c) the formation of compound (XII), 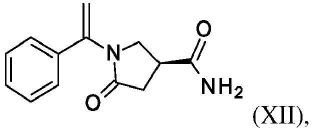 via elimination reaction of compound (XI);
step d) the formation of compound (XIII), 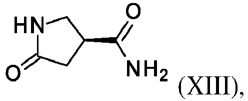 by deprotection of compound (XII);
step e) the formation of compound (IV), 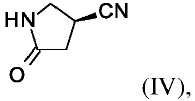 via dehydration of compound (XIII).

A detailed description of synthesis of compound (IV) in present invention of process steps is as following:
Step a) the formation of compound (X), 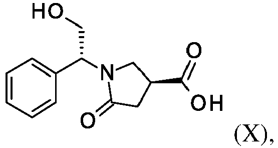 via cyclization reaction between compound (VIII), 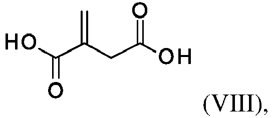 and compound (IX),

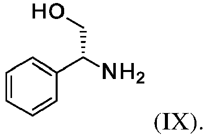

The formation of compound (X) is usually performed in the presence of a suitable organic solvent. The conversion as a rule is performed under a heating condition.

The suitable organic solvent is selected from THF, ACN and MeTHF, particularly the organic solvent is ACN.

The cyclization reaction as a rule is performed at 80~140 °C, particularly at 100~110 °C.

Step b) the formation of compound (XI), 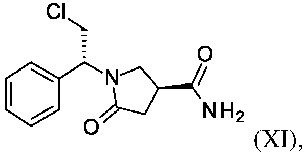 via substitution of compound (X).

The formation of compound (XI) is usually performed in the presence of a chlorination reagent and a suitable organic solvent. The conversion as a rule is performed under room temperature.

The suitable chlorination reagent is selected from acetyl chloride, thionyl chloride and oxalyl chloride, particularly the reagent is thionyl chloride, and the amount of the chlorination reagent is 1∼5 eq., particularly 3 eq.

The suitable organic solvent is selected from IPAc, DCM, Toluene, THF and MeTHF, particularly the organic solvent is DCM.

The substitution reaction as a rule is performed at 10~40 °C, particularly at 20~25 °C.

Step c) the formation of compound (XII), 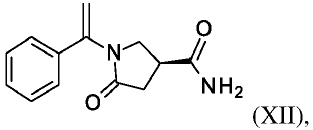 via elimination reaction of compound (XI);
The formation of compound (XII) is usually performed in the presence of a suitable base and a suitable solvent. The conversion as a rule is performed under a heating condition.

The suitable base is selected from K₃PO₄, TEA, DBU and DIPEA, particularly the base is DBU, and the amount of base is 1∼2 eq., particularly 1.5 eq.

The suitable solvent is selected from DCM, ACN, THF and Me-THF, particularly the solvent is THF.

The elimination reaction as a rule is performed at 20~80 °C, particularly at 40~45 °C.

Step d) the formation of compound (XIII), 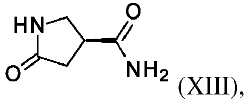 by deprotection of compound (XII);
The formation of compound (XIII) is usually performed in the presence of a suitable acid and a suitable organic solvent. The conversion as a rule is performed under room temperature.

The suitable acid is selected from H₂SO₄, H₃PO₄, HCl and TFA, particularly the acid is HCl, and the amount of acid is 0.05-0.5 eq., particularly 0.1 eq.

The suitable organic solvent is selected from EtOH, MeOH, IPA and IPAc, particularly the organic solvent is MeOH.

The deprotection reaction as a rule is performed under room temperature, particularly at 20~25 °C.

Step e) the formation of compound (IV), 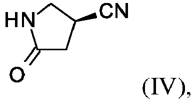 via dehydration of compound (XIII).

The formation of compound (IV) is usually performed in the presence of a suitable dehydration reagent and a suitable organic solvent. The conversion as a rule is performed under room temperature.

The suitable dehydration reagent is selected from P₂O₅, TFAA and acetic anhydride, particularly the dehydration reagent is TFAA, and the amount of dehydration reagent is 2.5 eq.

The suitable organic solvent is selected from EA, IPAc, MTBE and MeTHF, particularly the organic solvent is MTBE.

The dehydration reaction as a rule is performed under room temperature, particularly at 20~25 °C.

### EXAMPLES

### Example 1

### tert-butyl (6S)-3-iodo-6-methyl-6,7-dihydro-4H-pyrazolo[1,5-a]pyrazine-5-carboxylate (compound (III))

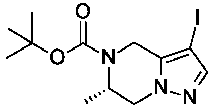

To a 1000 L jacket reactor was charged with tert-butyl (6S)-6-methyl-6,7-dihydro-4H-pyrazolo[1,5-a]pyrazine-5-carboxylate (14.1 kg, 59.5 mol, WuXi AppTec) and ACN (115 kg) at room temperature. To the mixture was then charged with NIS (20.0 kg, 88.9mol) at room temperature under N₂ protection. After being stirred at 20~30 °C for 18 hours, the reaction mixture was slowly cooled to -5-5 °C, and aqueous Na₂SO₃ solution (1.5 wt%, 4.3 kg) was added to quench the reaction. Then to the mixture was charged with water (140 kg) slowly over 1 hour at 5∼25 °C, the resulting mixture was stirred at 5∼25 °C for 1 h. The resulting suspension was separated via centrifuge in portions to collect wet cake. The wet cake was washed with ACN/water (22.3 kg, v/v=1/2.4) again, then dried using air oven (45~50 °C) for 48 hours to give the desired product as a white solid, which was directly used for the preparation of compound (V), giving 19.4 kg of tert-butyl (6S)-3-iodo-6-methyl-6,7-dihydro-4H-pyrazolo[1,5-a]pyrazine-5-carboxylate. The purity was 99.7 %, the yield was 90%. MS m/e = 363.2 [M+H]⁺. ¹H-NMR (400 MHz, DMSO-d⁶) δ = 7.78 (s, 1H), 4.73 (m, 1H), 4.54 (d, *J* = 16.8 Hz, 1H), 4.42 (dd, *J* = 12.1, 12.5 Hz, 1H), 4.07 (dd, *J* = 6.8, 12.1 Hz, 1H), 3.69 (d, *J* = 16.8 Hz, 1H), 1.53 (d, *J* = 6.6 Hz, 3H), 1.41 (s, 9H).

### Example 2

### (3S)-5-oxopyrrolidine-3-carbonitrile (compound (IV))

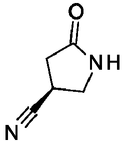

### Step a) synthesis of (3S)-5-oxo-1-[(1R)-2-hydroxy-1-phenyl-ethyl]pyrrolidine-3-carboxylic acid (compound (X))

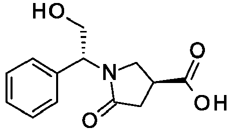

To a 1000 L jacket reactor was charged with Itaconic acid (62.5 kg, 480 mol, Qingdao Kehai Co. LTD), (R)-(-)-2-Phenylglycinol (70 kg, 510 mol, Jiangsu Senxuan pharmaceutical chemical co., LTD) and ACN (450 kg) at room temperature under N₂ atmosphere. The mixture was heated to 100~110 °C and the agitation was maintained for 5 hours. Then the mixture was cooled to 5∼10 °C and the agitation was maintained for 3 hours. The solid was filtered and washed with ACN (25 kg) to give the desired product as light yellow solid, which was directly used for the preparation of Compound XI, giving 31 kg of (3S)-5-oxo-1-[(1R)-2-hydroxy-1-phenyl-ethyl]pyrrolidine-3-carboxylic acid. The purity was 98.7 %, the chiral purity was 99.7%, the yield was 25 %, and the MS m/e = 250.1 [M+H]⁺. ¹H-NMR (400 MHz, DMSO-d⁶) δ 7.40 (d, 1H), 7.40 (d, 1H), 7.38 (dd, 1H), 7.38 (dd, 1H), 7.27 (t, 1H), 5.83 (dd, 1H), 4.05 (m, 1H), 3.98 (m, 1H), 3.58 (dd, 1H, *J*=8.5, 13.8 Hz), 3.39 (dd, 1H, *J*=8.5, 13.8 Hz), 3.15 (m, 1H), 2.71 (dd, 1H, *J*=9.4, 18.8 Hz), 2.51 (dd, 1H, *J*=9.3, 18.8 Hz).

### Step b) synthesis of (3S)-5-oxo-1-[(1R)-2-chloro-1-phenyl-ethyl]pyrrolidine-3-carboxamide (compound (XI))

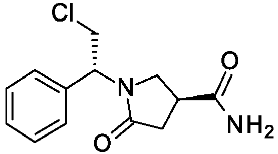

To a 500 L jacket reactor was charged with (3S)-5-oxo-1-[(1R)-2-hydroxy-1-phenylethyl]pyrrolidine-3-carboxylic acid (23.2 kg, 93.2 mol) and DCM (162.4 kg) at room temperature under N₂ atmosphere. To the stirred mixture was slowly charged with thionyl chloride (31 kg, 260.5 mol) at room temperature. The mixture was agitated at 20~25 °C for another 4 hours. The reaction mixture was concentrated *in vacuo* at 45 °C (0.09-0.1 MPa) until no solvent is distilled out. To the residue was charged with DCM (162.4 kg) and ammonia (7 kg, 410 mol) at below 20 °C. The mixture was stirred at 20~25 °C for 16 hours. To the mixture was charged with water (115 kg) and HOAc (3.5 kg) to adjust pH to 6-7. Then the mixture was concentrated under *vacuo* at 30~40 °C to evaporate DCM. Then the mixture was cooled to 10-15 °C and stirred at 10∼15 °C for 2 hours. The solid was filtered and washed with water (50 kg) to give the desired product as light yellow solid, which was directly used for the preparation of Compound XII, giving 22 kg of (3S)-5-oxo-1-[(1R)-2-chloro-1-phenyl-ethyl]pyrrolidine-3-carboxamide. The purity was 98 %, the chiral purity was 99.9%, the yield was 88 %, and the MS m/e = 267.1 [M+H]⁺. ¹H-NMR (400 MHz, DMSO-d⁶) δ 7.38 (dd, 1H, J=7.3, 7.4 Hz), 7.38 (dd, 1H, J=7.3, 7.4 Hz), 7.34 (d, 1H, J=7.4 Hz), 7.34 (d, 1H, J=7.4 Hz), 7.25 (t, 1H, J=7.3 Hz), 5.63 (dd, 1H, J=3.3, 8.3 Hz), 3.88 (dd, 1H, J=3.3, 12.4 Hz), 3.69 (dd, 1H, J=8.3, 12.4 Hz), 3.33 (dd, 1H, J=8.5, 13.8 Hz), 3.29 (dd, 1H, J=8.5, 13.8 Hz), 3.21 (m, 1H), 2.59 (dd, 1H, J=9.4, 18.8 Hz), 2.21 (dd, 1H, J=9.3, 18.8 Hz).

### Step c) synthesis of (3S)-5-oxo-1-(1-phenylvinyl)pyrrolidine-3-carboxamide (compound (XII))

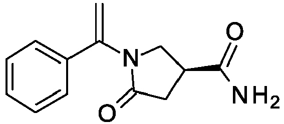

To a 200 L jacket reactor was charged with (3S)-5-oxo-1-[(1R)-2-chloro-1-phenylethyl]pyrrolidine-3-carboxamide (22 kg, 82.5 mol), THF (88 kg) and DBU (18.5 kg, 121.6 mol) at room temperature under N₂ atmosphere. The mixture was agitated using a magnetic stirrer at 40∼45 °C for 8 hours. Then the mixture was charged with HOAc (2.7 kg, 45.4 mol) to adjust pH to 6-7. The mixture was concentrated under vacuum at 40~45 °C to evaporate excess of THF. To the mixture was charged with water (40 kg) and the mixture was agitated at 10-15 °C for 3 hours. The solid was filtered and washed with water (25 kg) to give the desired product as light yellow solid, which was directly used for the preparation of Compound XIII, giving 15.4 kg of (3S)-5-oxo-1-(1-phenylvinyl)pyrrolidine-3-carboxamide. The purity was 99%, the chiral purity was 99.9%, the yield was 82 %, and the MS m/e = 231.1 [M+H]⁺. ¹H-NMR (400 MHz, DMSO-d⁶) δ 7.39 (t, 1H, J=7.3 Hz), 7.33 (dd, 1H, J=7.3, 7.4 Hz), 7.33 (dd, 1H, J=7.3, 7.4 Hz), 7.28 (d, 1H, J=7.4 Hz), 7.28 (d, 1H, J=7.4 Hz), 4.77 (s, 1H), 4.57 (s, 1H), 3.77 (dd, 1H, J=8.5, 13.8 Hz), 3.48 (dd, 1H, J=8.5, 13.8 Hz), 3.20 (m, 1H), 2.59 (dd, 1H, J=9.4, 18.8 Hz), 2.21 (dd, 1H, J=9.3, 18.8 Hz).

### Step d) synthesis of (3S)-5-oxopyrrolidine-3-carboxamide (compound (XIII))

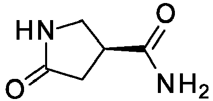

To a 500 L jacket reactor was charged with (3S)-5-oxo-1-(1-phenylvinyl)pyrrolidine-3-carboxamide (38.7 kg, 168 mol), methanol (108 kg) and hydrochloric acid (1.66 kg, 16.4 mol) at room temperature under N₂ atmosphere. The mixture was agitated using a magnetic stirrer at 20~25 °C for 4 hours. Then the mixture was cooled to 0∼5 °C, and agitated at that temperature for 3 hours. The solid was filtered and washed with methanol (40 kg) to give the desired product as off white solid, which was directly used for the preparation of Compound IV, giving 17 kg of (3S)-5-oxopyrrolidine-3-carboxamide. The purity was 99 %, the chiral purity was 100%, the yield was 80%, and the MS m/e = 129.1 [M+H]⁺. ¹H-NMR (400 MHz, DMSO-d⁶) δ 3.83 (dd, 1H, J=8.5, 13.8 Hz), 3.40 (dd, 1H, J=8.5, 13.8 Hz), 3.03 (m, 1H), 2.52 (dd, 1H, J=9.4, 18.8 Hz), 2.13 (dd, 1H, J=9.3, 18.8 Hz).

### Step e) synthesis of (3S)-5-oxopyrrolidine-3-carbonitrile (compound (IV))

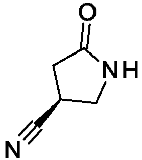

To a 200 L jacket reactor was charged with (3S)-5-oxopyrrolidine-3-carboxamide (17 kg, 132.7 mol) and MTBE (63 kg) at room temperature under N₂ atmosphere. The mixture was cooled to -5~0°C using Huber chiller. To the stirred mixture was charged with TFAA (70 kg, 333 mol) at -5~0°C. The mixture was agitated using a magnetic stirrer at 20~25°C for another 4 hours. Then the mixture was cooled to -5~0°C again. The solid was filtered and washed with MTBE (30 kg) to give the desired product as off white solid, which was directly used for the preparation of compound (V), giving 11.7 kg of (3S)-5-oxopyrrolidine-3-carbonitrile. The purity was 99.3 %, the chiral purity was 100%, the yield was 81 %.MS m/e = 110.1 [M+H]⁺. ¹H-NMR (400 MHz, DMSO-d⁶) δ = 3.71 (dd, *J* = 5.6, 9.2 Hz, 1H), 3.44 (dd, *J* = 7.5, 9.2 Hz, 1H), 3.32 (m, 1H), 2.97 (dd, *J* = 8.5, 18.2 Hz, 1H), 2.88 (dd, *J* = 8.4, 18.2 Hz, 1H).

### Example 3

### tert-Butyl (6S)-6-methyl-3-[(4S)-4-cyano-2-oxo-pyrrolidin-1-yl]-6,7-dihydro-4H-pyrazolo[1,5-a]pyrazine-5-carboxylate (compound (V))

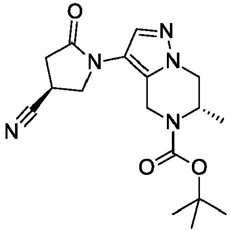

To a 100 L jacket reactor was charged with THF (36 L), K₂CO₃ (5.14 kg), tert-butyl (6S)-3-iodo-6-methyl-6,7-dihydro-4H-pyrazolo[1,5-a]pyrazine-5-carboxylate (4.5 kg, 12.4 mol), (3S)-5-oxopyrrolidine-3-carbonitrile (2.04 kg, 18.5 mol), CuI (236 g, 1.24 mol) and N,N'-Dimethyl-1,2-ethanediamine (218 g, 2.5 mol) at room temperature under N₂ atmosphere. The reaction mixture was heated to 75°C and the agitation was maintained for 1 hour. To the mixture was charged with another portion of N,N'-Dimethyl-1,2-ethanediamine (872 g, 10 mol) dropwise under refluxing. After being stirred under reflux for another 20 hours, the reaction mixture was slowly cooled to room temperature, and water (10 L) and IPAc (15 L) were added. After another 20 minutes, two layers were separated, and the organic layer was washed with 1N citric acid solution (6 L), 8% NaHCO₃ (8 L) solution and 10% brine (15 L). The organic layer was filtered through a Na₂SO₄ pad, and concentrated under reduced pressure to give the desired product as a light yellow solid, which was directly used for the preparation of compound (VI), giving 2.87 kg of tert-butyl (6S)-6-methyl-3-[(4S)-4-cyano-2-oxo-pyrrolidin-1-yl]-6,7-dihydro-4H-pyrazolo[1,5-a]pyrazine-5-carboxylate. The purity was 99 %, the yield was 67%, and the MS m/e = 345.2 [M+H]⁺. ¹H-NMR (400 MHz, DMSO-d⁶) δ = 7.33 (s, 1H), 4.71 (d, J = 16.8 Hz, 1H), 4.62 (m, 1H), 4.34 (dd, J = 12.1, 12.5 Hz, 1H), 4.05 (dd, J = 5.6, 9.2 Hz, 1H), 3.96 (dd, J = 6.8, 12.1 Hz, 1H), 3.86 (d, J = 16.8 Hz, 1H), 3.77 (dd, J = 7.5, 9.2 Hz, 1H), 3.44 (m, 1H), 3.22 (dd, J = 8.5, 18.2 Hz, 1H), 3.13 (dd, J = 8.4, 18.2 Hz, 1H), 1.52 (d, J = 6.6 Hz, 3H), 1.41 (s, 9H).

### Example 4

### (3S)-5-oxo-1-[(6S)-6-methyl-4,5,6,7-tetrahydropyrazolo[1,5-a]pyrazin-3-yl]pyrrolidine-3-carbonitrile (compound (VI))

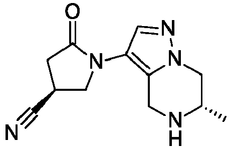

To a 1L flask was charged with (6S)-6-methyl-3-[(4S)-4-cyano-2-oxo-pyrrolidin-1-yl]-6,7-dihydro-4H-pyrazolo[1,5-a]pyrazine-5-carboxylate (124g, 359 mmol), HOAc (32.3 g, 539 mmol) and deionized water (620 mL) at room temperature. was After being stirred at 95 °C for 20 hours, the reaction mixture was cooled to room temperature and the water was azetroped with Toluene (300 mL×3). The resulting residue was triturated in MTBE (150 mL)/IPAc (150 mL) to give the desired product as a white solid, which was directly used for the preparation of compound (I), giving 1.6 kg of (3S)-5-oxo-1-[(6S)-6-methyl-4,5,6,7-tetrahydropyrazolo[1,5-a]pyrazin-3-yl]pyrrolidine-3-carbonitrile. The purity was 99.3%, the yield was 95%. MS m/e = 245.1 [M+H]⁺. ¹H-NMR (400 MHz, DMSO-d⁶) δ = 7.32 (s, 1H), 4.19 (dd, *J* = 11.1, 16.4 Hz, 1H), 4.06 (dd, *J* = 4.3, 16.4 Hz, 1H), 4.00 (dd, *J* = 5.6, 9.2 Hz, 1H), 3.96 (dd, *J* = 3.5, 12.4 Hz, 1H), 3.72 (dd, *J* = 7.5, 9.2 Hz, 1H), 3.63 (dd, *J* = 10.5, 12.4 Hz, 1H), 3.28 (m, 1H), 3.28 (m, 1H), 3.17 (dd, *J* = 8.5, 18.2 Hz, 1H), 3.08 (dd, *J* = 8.4, 18.2 Hz, 1H), 1.02 (d, *J =* 6.4 Hz, 3H).

### Example 5

### Phenyl N-(3,4,5-trifluorophenyl)carbamate (compound (VII))

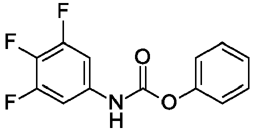

To a 20 L jacket reactor was charged with 3,4,5-trifluoroaniline (1 kg, 3.8 mol) and THF (10 L) at room temperature under N₂ atmosphere. The mixture was cooled to -5~0 °C. To the stirred mixture was charged with an aqueous solution of NaHCO₃ (856 g, 10.2 mol) in water (5 L) and phenyl carbonochloridate (1.2 kg, 7.48 mol) at -5~0 °C. After the addition, the reaction mixture was stirred at 0~10 °C for another 1 hour, then EtOAc (5 L) and water (2 L) were added. Two layers were separated, and the organic layer was washed with sat. NaCl (6 L), filtered through a Na₂SO₄ pad, and concentrated under reduced pressure to give the desired product as white solid, which was directly used for the preparation of compound (I), giving 0.9 kg of phenyl N-(3,4,5-trifluorophenyl)carbamate. The purity was 99%, the yield was 92%, and the MS m/e = 267.1 [M+H]⁺. ¹H-NMR (400 MHz, CDCl₃) δ = 7.43 (dd, J = 7.5, 8.1 Hz, 1H), 7.43 (dd, J = 7.5, 8.1 Hz, 1H), 7.28 (dd, J = 7.5, 12.3 Hz, 1H), 7.28 (dd, J = 7.5, 12.3 Hz, 1H), 7.20 (t, J = 7.5 Hz, 1H), 7.19 (dd, J = 2.7, 8.1 Hz, 1H), 7.19 (dd, J = 2.7, 8.1 Hz, 1H).

### Example 6

### (6S)-3-[(4S)-4-cyano-2-oxo-pyrrolidin-1-yl]-6-methyl-N-(3,4,5-trifluorophenyl)-6,7-dihydro-4H-pyrazolo[1,5-a]pyrazine-5-carboxamide (compound (I))

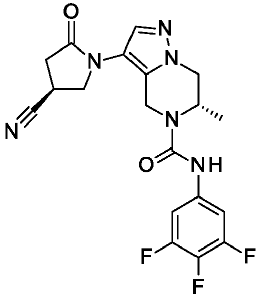

To a 50 L jacket reactor was charged with (3S)-5-oxo-1-[(6S)-6-methyl-4,5,6,7-tetrahydropyrazolo[1,5-a]pyrazin-3-yl]pyrrolidine-3-carbonitrile (2.2kg, 9 mol), phenyl N-(3,4,5-trifluorophenyl)carbamate (2.4 kg, 9 mol), IPAc (19.1 kg), acetone (3.3 kg) and DIPEA (1.39 kg ) under N₂ atmosphere. The mixture was agitated using a magnetic stirrer at 45~50 °C for 20 hours. Then the mixture was cooled to room temperature slowly. The white solid was filtered and washed with IPAc:MTBE (V/V=2/1, 6.7 kg). The wet cake was dried in a vacuum oven (IT = 50 °C) to give the desired product as a white solid, giving 3.5 kg of (6S)-3-[(4S)-4-cyano-2-oxopyrrolidin-1-yl]-6-methyl-N-(3,4,5-trifluorophenyl)-6,7-dihydro-4H-pyrazolo [1,5-a]pyrazine-5-carboxamide. The purity was 98.5%, the chiral purity was 100%, the yield was 93%. MS m/e = 418.1 [M+H]⁺. ¹H-NMR (400 MHz, DMSO-d⁶) δ = 7.38 (s, 1H), 7.16 (dd, J = 7.4, 13.4 Hz, 1H), 7.16 (dd, J = 7.4, 13.4 Hz, 1H), 4.43 (m, 1H), 4.29 (d, J = 16.8 Hz, 1H), 4.13 (dd, J = 12.1, 12.5 Hz, 1H), 3.99 (dd, J = 5.6, 9.2 Hz, 1H), 3.84 (d, J = 16.8 Hz, 1H), 3.80 (dd, J = 6.8, 12.1 Hz, 1H), 3.71 (dd, J = 7.5, 9.2 Hz, 1H), 3.44 (m, 1H), 3.22 (dd, J = 8.5, 18.2 Hz, 1H), 3.13 (dd, J = 8.4, 18.2 Hz, 1H), 1.31 (d, J = 6.6 Hz, 3H).

## Claims

1. Process for the preparation of a compound (I), 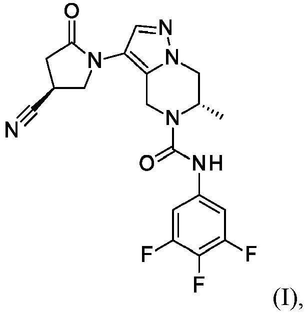
or pharmaceutically acceptable salt thereof;
comprising the following steps:
step 1) the formation of compound (III), 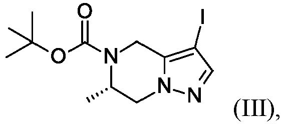 via iodization reaction of compound (II), 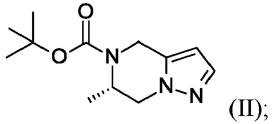
step 2) the formation of compound (V), 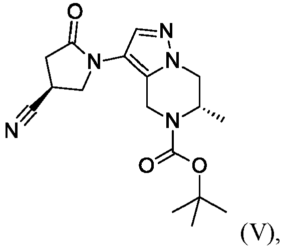 via Ullmann reaction between compound (III) and compound (IV), 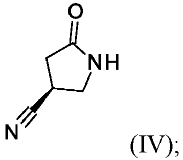
step 3) the formation of compound (VI), 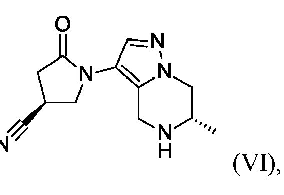 via de-protection of compound (V);
step 4) the formation of compound (VII), 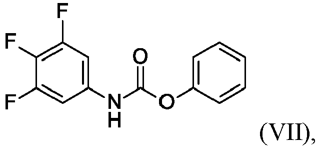 via the reaction between 3,4,5-trifluoroaniline and phenyl carbonochloridate;
step 5) the formation of compound (I), 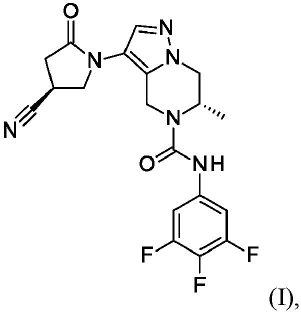 via the substitution reaction between compound (VI) and compound (VII).

2. A process according to claim 1, **characterized in that** the formation of compound (III) in step 1) is performed in the presence of an iodization reagent, wherein the iodization reagent is selected from NIS and I₂; particularly the iodization reagent is NIS.

3. A process according to claim 1 or 2, **characterized in that** the excessive I₂ formed during the reaction due to the use of iodization reagent in step 1) is removed by Na₂SO₃, wherein the amount of Na₂SO₃ is 0.4-0.8 eq., particularly 0.55-0.6 eq.

4. A process according to any one of claims 1 to 3, **characterized in that** the formation of compound (V) in step 2) is performed in the presence of a base, a catalyst and a ligand in an organic solvent; wherein the base is selected from K₂CO₃, K₃PO₄ and Cs₂CO₃, particularly the base is K₂CO₃; wherein the catalyst is CuI and the amount of catalyst is 0.05-0.5eq, particularly 0.1 eq.; wherein the ligand is DMEDA and the amount of ligand is 0.2-2.0 eq, particularly 1.0 eq.; wherein the organic solvent is selected from 1,4-Dioxane, ACN, Toluene, THF and MeTHF, particularly the organic solvent is THF.

5. A process according to any one of claims 1 to 4, **characterized in that** the formation of compound (VI) in step 3) is performed in the presence of an acid, wherein the acid is selected from H₃PO₄, NH₄Cl, TFA and acetic acid, particularly the acid is acetic acid; wherein the amount of acid is 1-3eq., particularly 1.5-2 eq.

6. A process according to any one of claims 1 to 5, **characterized in that** the formation of compound (VII) in step 4) is performed in the presence of a base, wherein the base is selected from K₂CO₃, KHCO₃, NaHCO₃ and Na₂CO₃, particularly the base is NaHCO₃.

7. A process according to claim 6, **characterized in that** the formation of compound (VII) in step 4) is performed at -10°C~10°C, particularly at -5-0 °C.

8. A process according to any one of claims 1 to 7, **characterized in that** the formation of compound (I) in step 5) is performed in the presence of a base, wherein the base is selected from K₂CO₃, K₃PO₄, DIPEA and TEA, particularly the base is DIPEA.

9. A process according to claim 1, further comprising the following steps:
step a) the formation of compound (X), 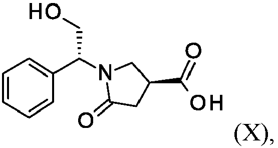 via cyclization reaction between compound (VIII), 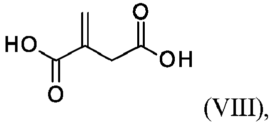 and compound (IX), 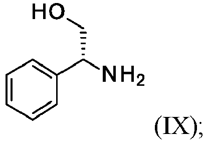
step b) the formation of compound (XI), 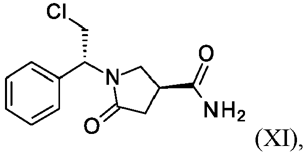 via substitution of compound (X);
step c) the formation of compound (XII), 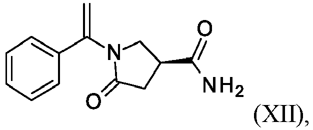 via elimination reaction of compound (XI);
step d) the formation of compound (XIII), 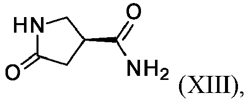 by deprotection of compound (XII);
step e) the formation of compound (IV), 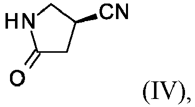 via dehydration of compound (XIII).

10. A process according to claim 9, **characterized in that** the formation of compound (X) in step a) is performed via cyclization reaction in an organic solvent, wherein the solvent is selected from THF, ACN and MeTHF, particularly the organic solvent is ACN; wherein the cyclization reaction is performed at 80~140 °C, particularly at 100~110 °C.

11. A process according to claim 9 or 10, **characterized in that** the formation of compound (XI) in step b) is performed in the presence of chlorination reagent, wherein the chlorination reagent is selected from acetyl chloride, thionyl chloride and oxalyl chloride, particularly the reagent is thionyl chloride; wherein the amount of the chlorination reagent is 1∼5 eq., particularly 3 eq.

12. A process according to any of claims 9 to 11, **characterized in that** the formation of compound (XII) in step c) is performed in the presence of a base, wherein the base is selected from K₃PO₄, TEA, DBU and DIPEA, particularly the base is DBU; wherein the amount of base is 1-2eq., particularly 1.5 eq.

13. A process according to any of claims 9 to 12, **characterized in that** the formation of compound (XIII) in step d) is performed in the presence of an acid, wherein the acid is selected from H₂SO₄, H₃PO₄, HCl and TFA, particularly the acid is HCl; wherein the amount of acid is 0.05-0.5eq., particularly 0.1 eq.

14. A process according to any of claims 9 to 13, **characterized in that** the formation of compound (IV) in step e) is performed in the presence of a dehydration reagent, wherein the dehydration reagent is selected from P₂O₅, TFAA and acetic anhydride, particularly the dehydration reagent is TFAA; wherein the amount of dehydration reagent is 2.5 eq.

## Patentansprüche

1. Verfahren zur Herstellung einer Verbindung (I) 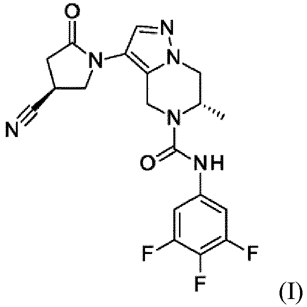
oder eines pharmazeutisch unbedenklichen Salzes davon;
umfassend die folgenden Schritte:
Schritt 1) die Bildung von Verbindung (III), 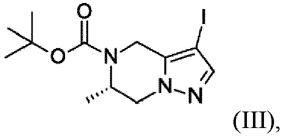 über eine Iodierungsreaktion von Verbindung (II), 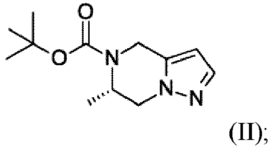
Schritt 2) die Bildung von Verbindung (V), 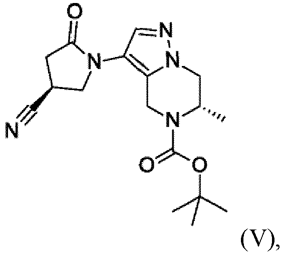 über eine Ullmann-Reaktion zwischen Verbindung (III) und Verbindung (IV), 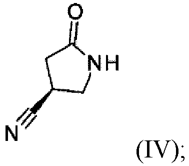
Schritt 3) die Bildung von Verbindung (VI), 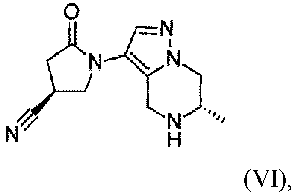 über eine Entschützung von Verbindung (V);
Schritt 4) die Bildung von Verbindung (VII), 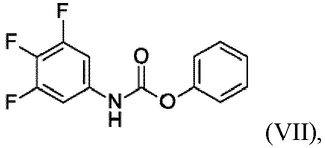 über die Reaktion zwischen 3,4,5-Trifluoranilin und Phenylcarbonochloridat;
Schritt 5) die Bildung von Verbindung (I), 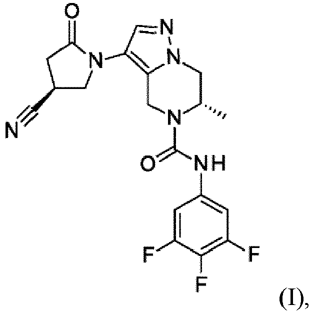 über die Substitutionsreaktion zwischen Verbindung (VI) und Verbindung (VII).

2. Verfahren nach Anspruch 1, **dadurch gekennzeichnet, dass** die Bildung von Verbindung (III) in Schritt 1) in der Gegenwart eines Iodierungsreagens durchgeführt wird, wobei das Iodierungsreagens aus NIS und I₂ ausgewählt ist; insbesondere das Iodierungsreagens NIS ist.

3. Verfahren nach Anspruch 1 oder 2, **dadurch gekennzeichnet, dass** das überschüssige I₂, das während der Reaktion aufgrund der Verwendung von Iodierungsreagens in Schritt 1) gebildet wird, mit Na₂SO₃ entfernt wird, wobei die Menge an Na₂SO₃ 0,4-0,8 Äqu., insbesondere 0,55-0,6 Äqu. beträgt.

4. Verfahren nach einem der Ansprüche 1 bis 3, **dadurch gekennzeichnet, dass** die Bildung von Verbindung (V) in Schritt 2) in der Gegenwart einer Base, eines Katalysators und eines Liganden in einem organischen Lösungsmittel durchgeführt wird; wobei die Base aus K₂CO₃, K₃PO₄ und Cs₂CO₃ ausgewählt ist, insbesondere die Base K₂CO₃ ist; wobei der Katalysator CuI ist und die Menge an Katalysator 0,05-0,5 Äqu., insbesondere 0,1 Äqu. beträgt; wobei der Ligand DMEDA ist und die Menge an Ligand 0,2-2,0 Äqu., insbesondere 1,0 Äqu. beträgt; wobei das organische Lösungsmittel aus 1,4-Dioxan, ACN, Toluol, THF und MeTHF ausgewählt ist, insbesondere das organische Lösungsmittel THF ist.

5. Verfahren nach einem der Ansprüche 1 bis 4, **dadurch gekennzeichnet, dass** die Bildung von Verbindung (VI) in Schritt 3) in der Gegenwart einer Säure durchgeführt wird, wobei die Säure aus H₃PO₄, NH₄Cl, TFA und Essigsäure ausgewählt ist, insbesondere die Säure Essigsäure ist; wobei die Menge an Säure etwa 1~3 Äqu., insbesondere etwa 1,5~2 Äqu. beträgt.

6. Verfahren nach einem der Ansprüche 1 bis 5, **dadurch gekennzeichnet, dass** die Bildung von Verbindung (VII) in Schritt 4) in der Gegenwart einer Base durchgeführt wird, wobei die Base aus K₂CO₃, KHCO₃, NaHCO₃ und Na₂CO₃ ausgewählt ist, insbesondere die Base NaHCO₃ ist.

7. Verfahren nach Anspruch 6, **dadurch gekennzeichnet, dass** die Bildung von Verbindung (VII) in Schritt 4) bei etwa -10 °C~10 °C, insbesondere bei etwa -5~0 °C durchgeführt wird.

8. Verfahren nach einem der Ansprüche 1 bis 7, **dadurch gekennzeichnet, dass** die Bildung von Verbindung (I) in Schritt 5) in der Gegenwart einer Base durchgeführt wird, wobei die Base aus K₂CO₃, K₃PO₄, DIPEA und TEA ausgewählt ist, insbesondere die Base DIPEA ist.

9. Verfahren nach Anspruch 1, ferner umfassend die folgenden Schritte:
Schritt a) die Bildung von Verbindung (X), 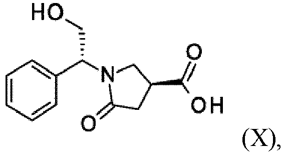 über eine Zyklisierungsreaktion zwischen Verbindung (VIII), 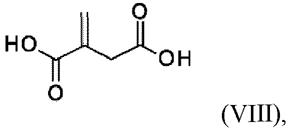 und Verbindung (IX), 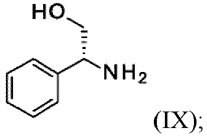
Schritt b) die Bildung von Verbindung (XI), 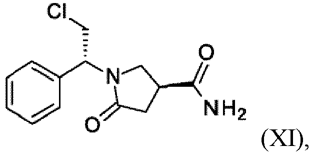 über eine Substitution von Verbindung (X);
Schritt c) die Bildung von Verbindung (XII), 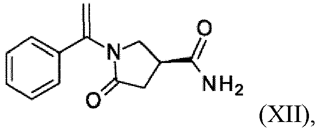 über eine Eliminierungsreaktion von Verbindung (XI);
Schritt d) die Bildung von Verbindung (XIII), 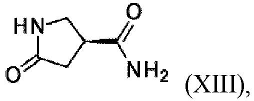 durch eine Entschützung von Verbindung (XII);
Schritt e) die Bildung von Verbindung (IV), 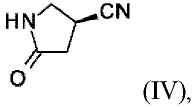 über eine Dehydratisierung von Verbindung (XIII).

10. Verfahren nach Anspruch 9, **dadurch gekennzeichnet, dass** die Bildung von Verbindung (X) in Schritt a) über eine Zyklisierungsreaktion in einem organischen Lösungsmittel durchgeführt wird, wobei das Lösungsmittel aus THF, ACN und MeTHF ausgewählt ist, insbesondere das organische Lösungsmittel ACN ist; wobei die Zyklisierungsreaktion bei etwa 80∼140 °C, insbesondere bei etwa 100~110 °C durchgeführt wird.

11. Verfahren nach Anspruch 9 oder 10, **dadurch gekennzeichnet, dass** die Bildung von Verbindung (XI) in Schritt b) in der Gegenwart eines Chlorierungsreagens durchgeführt wird, wobei das Chlorierungsreagens aus Acetylchlorid, Thionylchlorid und Oxalylchlorid ausgewählt ist, insbesondere das Reagens Thionylchlorid ist; wobei die Menge an dem Chlorierungsreagens etwa 1∼5 Äqu., insbesondere 3 Äqu. beträgt.

12. Verfahren nach einem der Ansprüche 9 bis 11, **dadurch gekennzeichnet, dass** die Bildung von Verbindung (XII) in Schritt c) in der Gegenwart einer Base durchgeführt wird, wobei die Base aus K₃PO₄, TEA, DBU und DIPEA ausgewählt ist, insbesondere die Base DBU ist; wobei die Menge an Base etwa 1∼2 Äqu., insbesondere 1,5 Äqu. beträgt.

13. Verfahren nach einem der Ansprüche 9 bis 12, **dadurch gekennzeichnet, dass** die Bildung von Verbindung (XIII) in Schritt d) in der Gegenwart einer Säure durchgeführt wird, wobei die Säure aus H₂SO₄, H₃PO₄, HCl und TFA ausgewählt ist, insbesondere die Säure HCl ist; wobei die Menge an Säure etwa 0,05-0,5 Äqu., insbesondere 0,1 Äqu. beträgt.

14. Verfahren nach einem der Ansprüche 9 bis 13, **dadurch gekennzeichnet, dass** die Bildung von Verbindung (IV) in Schritt e) in der Gegenwart eines Dehydratisierungsreagens durchgeführt wird, wobei das Dehydratisierungsreagens aus P₂O₅, TFAA und Essigsäureanhydrid ausgewählt ist, insbesondere das Dehydratisierungsreagens TFAA ist; wobei die Menge an Dehydratisierungsreagens 2,5 Äqu. beträgt.

## Revendications

1. Procédé de préparation d'un composé (I), 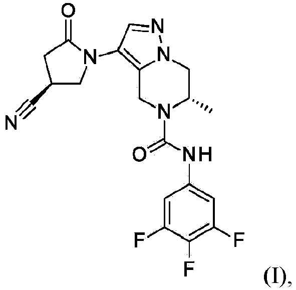
ou d'un sel pharmaceutiquement acceptable de celui-ci ;
comprenant les étapes suivantes :
étape 1) la formation du composé (III), 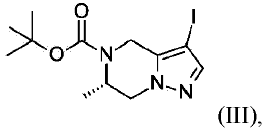 par le biais d'une réaction d'iodation du composé (II), 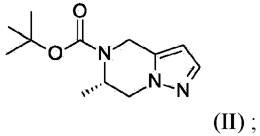
étape 2) la formation du composé (V), 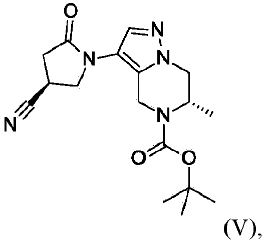 par le biais d'une réaction d'Ullmann entre le composé (III) et le composé (IV), 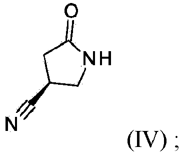
étape 3) la formation du composé (VI), 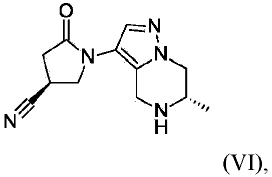 par le biais d'une déprotection du composé (V) ;
étape 4) la formation du composé (VII), 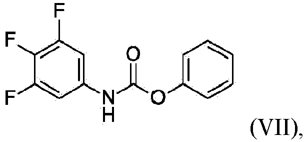 par le biais de la réaction entre la 3,4,5-trifluoroaniline et le carbonochloridate de phényle ;
étape 5) la formation du composé (I), 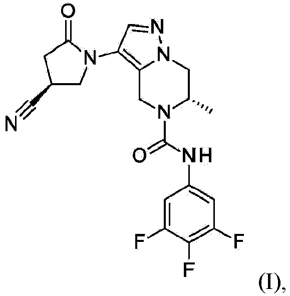 par le biais de la réaction de substitution entre le composé (VI) et le composé (VII).

2. Procédé selon la revendication 1, **caractérisé en ce que** la formation du composé (III) à l'étape 1) est réalisée en présence d'un réactif d'iodation, dans lequel le réactif d'iodation est choisi parmi NIS et I₂ ; en particulier le réactif d'iodation est NIS.

3. Procédé selon la revendication 1 ou 2, **caractérisé en ce que** l'excès d'I₂ formé pendant la réaction à cause de l'utilisation du réactif d'iodation à l'étape 1) est éliminé par Na₂SO₃, dans lequel la quantité de Na₂SO₃ est de 0,4 à 0,8 éq., en particulier de 0,55 à 0,6 éq.

4. Procédé selon l'une quelconque des revendications 1 à 3, **caractérisé en ce que** la formation du composé (V) à l'étape 2) est réalisée en présence d'une base, d'un catalyseur et d'un ligand dans un solvant organique ; dans lequel la base est choisie parmi K₂CO₃, K₃PO₄ et Cs₂CO₃, en particulier la base est K₂CO₃ ; dans lequel le catalyseur est CuI et la quantité de catalyseur est de 0,05 à 0,5 éq, en particulier 0,1 éq. ; dans lequel le ligand est la DMEDA et la quantité de ligand est de 0,2 à 2,0 éq., en particulier 1,0 éq. ; dans lequel le solvant organique est choisi parmi le 1,4-dioxane, l'ACN, le toluène, le THF et le MeTHF, en particulier le solvant est le THF.

5. Procédé selon l'une quelconque des revendications 1 à 4, **caractérisé en ce que** la formation du composé (VI) à l'étape 3) est réalisée en présence d'un acide, dans lequel l'acide est choisi parmi l'H₃PO₄, le NH₄Cl, le TFA et l'acide acétique, en particulier l'acide est l'acide acétique ; dans lequel la quantité d'acide est de 1∼3 éq., en particulier de 1,5~2 éq.

6. Procédé selon l'une quelconque des revendications 1 à 5, **caractérisé en ce que** la formation du composé (VII) à l'étape 4) est réalisée en présence d'une base, dans lequel la base est choisie parmi K₂CO₃, KHCO₃, NaHCO₃ et Na₂CO₃, en particulier la base est NaHCO₃.

7. Procédé selon la revendication 6, **caractérisé en ce que** la formation du composé (VII) à l'étape 4) est réalisée à -10°C~10°C, en particulier à -5~0 °C.

8. Procédé selon l'une quelconque des revendications 1 à 7, **caractérisé en ce que** la formation du composé (I) à l'étape 5) est réalisée en présence d'une base, dans lequel la base est choisie parmi K₂CO₃, K₃PO₄, DIPEA et TEA, en particulier la base est la DIPEA.

9. Procédé selon la revendication 1, comprenant en outre les étapes suivantes :
étape a) la formation du composé (X), 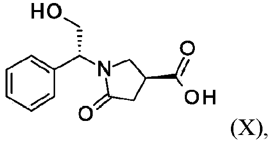 par le biais d'une réaction de cyclisation entre le composé (VIII), 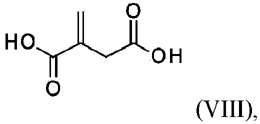 et le composé (IX), 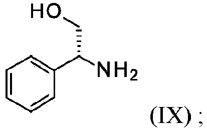
étape b) la formation du composé (XI), 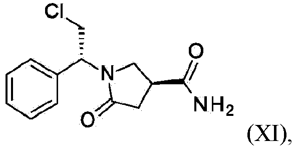 par le biais d'une substitution du composé (X) ;
étape c) la formation du composé (XII), 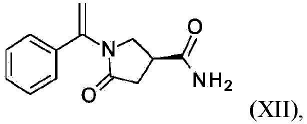 par le biais d'une réaction d'élimination du composé (XI) ;
étape d) la formation du composé (XIII), 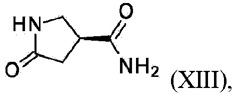 par déprotection du composé (XII) ;
étape e) la formation du composé (IV), 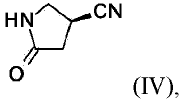 par le biais d'une déshydratation du composé (XIII).

10. Procédé selon la revendication 9, **caractérisé en ce que** la formation du composé (X) à l'étape a) est réalisée par le biais d'une réaction de cyclisation dans un solvant organique, dans lequel le solvant est choisi parmi le THF, l'ACN et le MeTHF, en particulier le solvant organique est l'ACN ; dans lequel la réaction de cyclisation est réalisée à 80∼140 °C, en particulier à 100∼110 °C.

11. Procédé selon la revendication 9 ou 10, **caractérisé en ce que** la formation du composé (XI) à l'étape b) est réalisée en présence d'un réactif de chloration, dans lequel le réactif de chloration est choisi parmi le chlorure d'acétyle, le chlorure de thionyle et le chlorure d'oxalyle, en particulier le réactif est le chlorure de thionyle ; dans lequel la quantité du réactif de chloration est de 1∼5 éq., plus particulièrement de 3 éq.

12. Procédé selon l'une quelconque des revendications 9 à 11, **caractérisé en ce que** la formation du composé (XII) à l'étape c) est réalisée en présence d'une base, dans lequel la base est choisie parmi le K₃PO₄, le TEA, le DBU et la DIPEA, en particulier la base est le DBU ; dans lequel la quantité de base est de 1∼2 éq., en particulier de 1,5 éq.

13. Procédé selon l'une quelconque des revendications 9 à 12, **caractérisé en ce que** la formation du composé (XIII) à l'étape d) est réalisée en présence d'un acide, dans lequel l'acide est choisi parmi l'H₂SO₄, l'H₃PO₄, l'HCl et le TFA, en particulier l'acide est l'HCl ; dans lequel la quantité d'acide est 0,05-0,5 éq., en particulier de 0,1 éq.

14. Procédé selon l'une quelconque des revendications 9 à 13, **caractérisé en ce que** la formation du composé (IV) à l'étape e) est réalisée en présence d'un réactif de déshydratation, dans lequel le réactif de déshydratation est choisi parmi le P₂O₅, le TFAA et l'anhydride acétique, en particulier le réactif de déshydratation est le TFAA ; dans lequel la quantité de réactif de déshydratation est de 2,5 éq.
